(19) Europäisches Patentamt
European Patent Office
Office européen des brevets

(11) **EP 3 542 641 B1**

(12) # EUROPÄISCHE PATENTSCHRIFT

(45) Veröffentlichungstag und Bekanntmachung des Hinweises auf die Patenterteilung:
**12.07.2023 Patentblatt 2023/28**

(21) Anmeldenummer: **18162827.2**

(22) Anmeldetag: **20.03.2018**

(51) Internationale Patentklassifikation (IPC):
**A23L 2/52** (2006.01)    **A23C 21/00** (2006.01)
**A23L 2/60** (2006.01)    **A61K 31/7016** (2006.01)
**C13K 13/00** (2006.01)    **A23L 33/125** (2016.01)

(52) Gemeinsame Patentklassifikation (CPC):
**A23L 2/52; A23C 9/1206; A23C 9/1422; A23C 9/1427; A23C 21/023; A23L 33/125; A61K 31/7016; A61P 1/14; C13K 13/005**

(54) **LACTULOSEHALTIGES PRODUKT SOWIE VERFAHREN ZUR HERSTELLUNG EINES LACTULOSEHALTIGEN PRODUKTS**

LACTULOSE-CONTAINING PRODUCT AND METHOD FOR PRODUCING A LACTULOSE-CONTAINING PRODUCT

PRODUIT CONTENANT DU LACTULOSE AINSI QUE PROCÉDÉ DE FABRICATION D'UN PRODUIT CONTENANT DU LACTULOSE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**25.09.2019 Patentblatt 2019/39**

(73) Patentinhaber:
 • **Universität Hohenheim**
  **70599 Stuttgart (DE)**
 • **CuraProducts GmbH**
  **64521 Gross-Gerau (DE)**

(72) Erfinder:
 • **Hinrichs, Jörg**
  **70599 Stuttgart (DE)**
 • **Nedele, Christian**
  **72124 Pliezhausen (DE)**
 • **Rauber, Michael**
  **79102 Freiburg (DE)**
 • **Krause, Lothar**
  **64846 Groß-Zimmern (DE)**

(74) Vertreter: **Patentanwälte**
 **Ruff, Wilhelm, Beier, Dauster & Partner mbB**
 **Kronenstraße 30**
 **70174 Stuttgart (DE)**

 • **WANG HE ET AL: "Enzymatic production of lactulose and 1-lactulose: current state and perspectives", APPLIED MICROBIOLOGY AND BIOTECHNOLOGY, SPRINGER, DE, Bd. 97, Nr. 14, 18. Juni 2013 (2013-06-18) , Seiten 6167-6180, XP035328670, ISSN: 0175-7598, DOI: 10.1007/S00253-013-4998-3 [gefunden am 2013-06-18]**
 • **SCHUSTER-WOLFF-BUEHRING R ET AL: "A new way to refine lactose: enzymatic synthesis of prebiotic lactulose", EUROPEAN DAIRY MAGA, GELSENKIRCHEN, DE, Nr. 7, 1. Januar 2009 (2009-01-01), Seiten 25-27, XP009142428, ISSN: 0936-6318**
 • **Anonymous: "Enzymatische Gewinnung von lactulose in lactulosehaltigen Milchprodukten und technischen Lactuloselösungen", , 1. Januar 2008 (2008-01-01), Seiten 1-3, XP055478247, Germany Gefunden im Internet: URL:file:///C:/Users/DB52148/AppData/Local /Temp/14787+kurz.pdf [gefunden am 2018-05-24]**
 • **KARIN FRSTER-FROMME ET AL: "A new enzymatically produced 1-lactulose: A pilot study to test the bifidogenic effects", INTERNATIONAL DAIRY JOURNAL, ELSEVIER APPLIED SCIENCE, BARKING, GB, Bd. 21, Nr. 12, 1. Juli 2011 (2011-07-01), Seiten 940-948, XP028293553, ISSN: 0958-6946, DOI: 10.1016/J.IDAIRYJ.2011.07.002 [gefunden am 2011-07-30]**

(56) Entgegenhaltungen:
 **WO-A1-2014/141164    CN-A- 102 180 913**
 **KR-A- 20130 101 690**

EP 3 542 641 B1

- DATABASE GNPD [Online] MINTEL; September 2016 (2016-09), Anonymous: "Peach Yogurt with Lactulose", XP002781384, Database accession no. 4294547
- DATABASE GNPD [Online] MINTEL; Oktober 2016 (2016-10), Anonymous: "Classic Yogurt with Digestive Formula", XP002781385, Database accession no. 4381541
- SCHUSTER-WOLFF-BUHRING R ET AL: "Production and physiological action of the disaccharide lactulose", INTERNATIONAL DAIRY JOURNAL, ELSEVIER APPLIED SCIENCE, BARKING, GB, Bd. 20, Nr. 11, 1. November 2010 (2010-11-01), Seiten 731-741, XP027198384, ISSN: 0958-6946 [gefunden am 2010-06-12]
- XIAO HUA ET AL: "Production of 1-lactulose and lactulose using commercial [beta]-galactosidase from Kluyveromyces lactis in the presence of fructose", FOOD CHEMISTRY, Bd. 137, Nr. 1-4, 12. Oktober 2012 (2012-10-12), Seiten 1-7, XP055478263, NL ISSN: 0308-8146, DOI: 10.1016/j.foodchem.2012.10.003
- AMARA AÏT-AISSA ET AL: "Lactulose: production and use in functional food, medical and pharmaceutical applications. Practical and critical review", INTERNATIONAL JOURNAL OF FOOD SCIENCE AND TECHNOLOGY., Bd. 49, Nr. 5, 30. Dezember 2013 (2013-12-30), Seiten 1245-1253, XP055478273, GB ISSN: 0950-5423, DOI: 10.1111/ijfs.12465

**Beschreibung**

ANWENDUNGSGEBIET UND STAND DER TECHNIK

Die Erfindung betrifft ein Verfahren zum Herstellen eines lactulosehaltigen Produkts sowie ein nach dem Verfahren hergestelltes oder herstellbares lactulosehaltiges Produkt.

Lactulose ist ein Disaccharid, welches aus D-Galactose und Fructose besteht. Lactulose ist durch eine als De-Bruyn-van-Ekenstein-Umlagerung bezeichnete Isomerisierung in alkalischer Lösung oder bei hohen Temperaturen aus Lactose (Milchzucker) herstellbar. Eine andere Möglichkeit ist die enzymatisch induzierte (biotechnologische) Herstellung mittels Transgalactosylierung.

Lactulose wird von Darmbakterien, darunter hauptsächlich Milchsäurebakterien und Bifidobakterien, teilweise zu niedermolekularen Fettsäuren, Wasserstoff und Methan abgebaut bzw. vergoren. Dies wird auch als bifidogene oder präbiotische Wirkung der Lactulose bezeichnet, da hierdurch das Wachstum dieser Bakterien verstärkt werden kann. Durch die entstandenen Säuren und die Zunahme der bakteriellen Masse im Dickdarm wird die Peristaltik angeregt und die abführende Wirkung von Lactulose verstärkt.

Präbiotische Produkte sowie biotechnologische Verfahren zu deren Herstellung sind bekannt. Beispielsweise ist aus der WO 2008/037839 A1 ein milchbasiertes Produkt, welches verschiedene Galactooligosaccharide, u.a. geringe Mengen an Lactulose, enthält, bekannt.

Weiterhin sind oligosaccharidhaltige Ernährungszusammensetzungen zur Kontrolle von entzündlichen Darmerkrankungen, wie beispielsweise Durchfall und Verstopfung, aus der US 8,241,658 B2 bekannt. Die US 9,579,340 B2 offenbart präbiotische Zusammensetzungen, welche Galactooligosaccharide enthalten, zur Behandlung von Symptomen, welche mit Laktoseintoleranz einhergehen.

Ferner sind oligosaccharidhaltige Ernährungszusammensetzungen oder pharmazeutische Zusammensetzungen zur Inhibierung bakterieller Adhäsion an Säugetierzellen aus der US 7,842,678 B2 bekannt.

Ferner ist die enzymatische Herstellung von Lactulose ausgehend von einem lactosehaltigen Permeat, welches durch Ultrafiltration aus Molke gewonnen wird, bekannt (Wang He et al.: "Enzymatic production of lactulose and 1-lactulose: current state and perspectives", Appl Microbiol Biotechnol (2013) 97:6167-6180).

Ferner ist aus der WO 2014/141164 A1 ein Verfahren zur Gewinnung von Lactose ausgehend von einer lactosehaltigen Flüssigkeit bekannt, wobei zur verbesserten Entfernung von Asche eine Diafiltration vor einer Nanofiltration durchgeführt wird.

Ein Nachteil von aus dem Stand der Technik bekannten Produkten ist, dass sie häufig lediglich einen geringen Lactuloseanteil besitzen und mithin eine präbiotische Wirkung nicht oder nur unzureichend zur Geltung kommt. Ein weiterer Nachteil besteht darin, dass aus dem Stand der Technik bekannte Präbiotika meistens einen hohen Gehalt an Monosacchariden und anderen Disacchariden (als Lactulose) aufweisen, was die Süße und insbesondere den Einfluss auf den glykämischen Index sowie den Energiegehalt der Produkte stark erhöht.

AUFGABE UND LÖSUNG

Die Erfindung stellt sich daher die Aufgabe, ein Verfahren zur Herstellung eines lactulosehaltigen Produkts, insbesondere mit präbiotischer Wirkung, sowie ein entsprechendes Produkt bereitzustellen, welche im Zusammenhang von gattungsgemäßen Präbiotika bekannte Nachteile, insbesondere in Bezug auf eine unzureichende präbiotische Wirkung und/oder eine übermäßige Süße und/oder einen hohen Energiegehalt, vermeiden.

Diese Aufgabe wird gelöst durch ein Verfahren gemäß unabhängigem Anspruch 1 sowie durch ein Produkt gemäß Anspruch 13. Bevorzugte Ausgestaltungen des Verfahrens sind in den abhängigen Ansprüchen 2 bis 12 sowie in der Beschreibung definiert. Bevorzugte Ausgestaltungen des Produkts sind in der Beschreibung definiert. Der Wortlaut sämtlicher Ansprüche wird hiermit durch ausdrückliche Bezugnahme zum Inhalt der Beschreibung gemacht.

Gemäß einem ersten Aspekt betrifft die Erfindung ein Verfahren zum Herstellen eines lactulosehaltigen Produkts, vorzugsweise eines lactulosehaltigen, präbiotischen Produkts.

Bei dem lactulosehaltigen Produkt kann es sich grundsätzlich um ein Lebensmittelprodukt, insbesondere um ein Getränk, oder um ein pharmazeutisches Produkt, insbesondere in Form eines lactulosehaltigen Konzentrats, handeln. Bevorzugt handelt es sich bei dem lactulosehaltigen Produkt um ein präbiotisches Lebensmittelprodukt, insbesondere um ein präbiotisches Getränk. Bei dem Konzentrat kann es sich insbesondere um einen Sirup, d.h. um eine dickflüssige, konzentrierte Lösung, handeln.

Bevorzugt handelt es sich bei dem lactulosehaltigen Produkt um ein präbiotisches Lebensmittelprodukt, insbesondere um ein präbiotisches Milch- oder Molkenprodukt, bevorzugt um ein präbiotisches Milch- oder Molkengetränk. Bei dem Molkenprodukt kann es sich um ein Sauermolkenprodukt, Süßmolkenprodukt oder um ein Molkenmischprodukt, d. h. um ein aus Sauermolke und Süßmolke hergestelltes Produkt, handeln. Weiterhin kann das Molkenprodukt einen sauren bis neutralen pH-Wert aufweisen. Bei dem Molkengetränk kann es sich um ein Sauermolkengetränk, Süßmolkengetränk oder Molkenmischgetränk, d.h. um ein aus Sauer- und Süßmolke hergestelltes Getränk, handeln.

Das Verfahren der vorliegenden Erfindung weist die folgenden Schritte auf:

a) Bereitstellen eines lactosehaltigen Substrats in Form eines lactosehaltigen Permeats, wobei es sich bei dem lactosehaltigen Permeat um ein Permeat von Milch, von Molke oder eines Milch- oder Molkenderivats handelt,

b) Behandeln des lactosehaltigen Substrats mit einer β-Galactosidase und/oder einer β-Glucosidase in Gegenwart von Fructose unter Erzeugung eines lactulosehaltigen Substrats, wobei die Fructose dem lactosehaltigen Substrat vor Durchführen von Schritt b) zugegeben wird,

c) Inaktivieren der β-Galactosidase und/oder der β-Glucosidase durch Erhitzen und

d) Erzeugen eines lactulosehaltigen Retentats durch Nanofiltration und Diafiltration des lactulosehaltigen Substrats, wobei die Diafiltration nach der Nanofiltration durchgeführt, die Nanofiltration bei einer transmembranen Druckdifferenz von 10 bar bis 50 bar durchgeführt und die transmembrane Druckdifferenz vor dem Diafiltrieren vermindert wird.

Unter dem Ausdruck "Milchderivat" soll im Sinne der vorliegenden Erfindung ein Weiterverarbeitungsprodukt von Milch, wie beispielsweise Milchpulver oder eine Milchpulverlösung, verstanden werden.

Unter dem Ausdruck "Molkenderivat" soll im Sinne der vorliegenden Erfindung ein Weiterverarbeitungsprodukt von Molke, insbesondere von Sauer- oder Süßmolke oder eines Gemisches aus Sauer- und Süßmolke, verstanden werden. Bei dem Molkenderivat kann es sich beispielsweise um Sauermolkenpulver, Süßmolkenpulver, ein Pulvergemisch aus Sauer- und Süßmolke, eine sauermolkenhaltige Lösung, eine süßmolkenhaltige Lösung oder eine sauer- und süßmolkenhaltige Lösung handeln.

Unter dem Ausdruck "lactulosehaltiges Konzentrat" soll im Sinne der vorliegenden Erfindung ein Pulver oder eine Flüssigkeit mit einem Anteil an Lactulose von wenigstens 10 Gew.-%, insbesondere 10 Gew.-% bis 80 Gew.-%, bevorzugt 60 Gew.-% bis 70 Gew.-%, bezogen auf das Gesamtgewicht des Pulvers bzw. der Flüssigkeit, verstanden werden.

Unter dem Ausdruck "ältere Personen" soll im Sinne der vorliegenden Erfindung Personen mit einem Alter von 50 Jahren oder mehr, insbesondere von 60 Jahren oder mehr, bevorzugt 70 Jahren oder mehr, verstanden werden.

Durch das erfindungsgemäße Verfahren lassen sich insbesondere die nachfolgenden Vorteile realisieren:

- Aufgrund des erfindungsgemäß vorgesehenen enzymatischen Behandlungsschritts (Schritt b)) ist eine reaktions-, regio- sowie stereoselektive Erzeugung von Lactulose unter milden Reaktionsbedingungen möglich.

- Durch den erfindungsgemäß vorgesehenen Inaktivierungsschritt (Schritt c)) kann mit besonderem Vorteil ein (nennenswerter) durch die β-Galactosidase und/oder β-Glucosidase katalysierter Abbau der in Schritt b) erzeugten Lactulose vermieden werden. Dies ermöglicht oder erleichtert eine nachgeschaltete Lactuloseanreicherung (Schritt d)).

- Durch Schritt d) findet mit besonderem Vorteil eine Anreicherung von Lactulose und gleichzeitig eine Abreicherung, insbesondere Auswaschung, von Monosacchariden und/oder anderen Disacchariden (als Lactulose) statt. Durch die Anreicherung von Lactulose gemäß Schritt d) kann ein Produkt hergestellt werden, welches Lactulose in einer insbesondere für die Ernährung von Frauen und/oder älteren und/oder pflegebedürftigen und/oder rehabilitationsbedürftigen Personen, welche insbesondere unter Verdauungsstörungen leiden oder bei welchen derartige Störungen ernstlich zu befürchten sind, ausreichenden präbiotischen Dosis enthält.

- Ein weiterer Vorteil der Lactuloseanreicherung besteht darin, dass zur Erzielung einer ausreichenden präbiotischen Wirkung geringere Mengen des lactulosehaltigen Produkts konsumiert werden müssen. Dies fördert allgemein die Akzeptanz und Verzehrbereitschaft auf Seiten der Konsumenten.

- Durch die beim Durchführen von Schritt d) gleichzeitig stattfindende Abreicherung von Monosacchariden und/oder anderen Disacchariden (als Lactulose) wird ferner mit besonderem Vorteil verhindert, dass das herzustellende Produkt eine übermäßige Süße besitzt. Dadurch wird die Akzeptanz und Verzehrbereitschaft auf Seiten der Konsumenten zusätzlich gestärkt. Durch eine reduzierte Süße des lactulosehaltigen Produkts kann außerdem ein nachteiliger Einfluss auf den glykämischen Index vermieden werden. Das lactulosehaltige Produkt kann daher insbesondere auch für die Ernährung von Personen, insbesondere älteren Personen, verwendet werden.

- Ein weiterer Vorteil des erfindungsgemäßen Verfahrens besteht darin, dass eine hochwertige Verwertungsmöglichkeit für Molke, insbesondere Sauermolke, geschaffen wird. Insbesondere Sauermolke stellt ein bislang kaum verwertbares Nebenprodukt bei der Milchherstellung dar und findet allenfalls Verwendung als Tierfutter oder als Substrat in Biogasanlagen.

- Ein weiterer Vorteil besteht darin, dass mittels des erfindungsgemäßen Verfahrens eine weitgehende Umsetzung von Lactose in Lactulose erzielt werden kann.

Das lactosehaltige Substrat wird durch Schritt a) in Form eines lactosehaltigen Permeats bereitgestellt. Bei dem lactosehaltigen Permeat handelt es sich um ein Permeat von Milch wie Magermilch, von Molke wie Sauer-, Süßmolke oder eines Gemisches von Sauer- und Süßmolke oder um ein Permeat eines Milch- oder Molkenderivats. Bevorzugt wird das lactosehaltige Substrat durch Schritt a) in Form eines Milchpermeats, eines Permeats einer milchhaltigen Lösung wie Milchpulverlösung, eines Sauermolkenpermeats, eines Permeats einer sauermolkenhaltigen Lösung wie einer Sauermolkenpulverlösung, eines Süßmolkenpermeats, eines Permeats einer süßmolkenhaltigen Lösung wie einer Süßmolkenpulverlösung oder eines Permeats einer sauer- und süßmolkenhaltigen Lösung wie einer sauermolkenpulver- und süßmolkenpulverhaltigen Lösung bereitgestellt.

In weiterer Ausgestaltung der Erfindung wird das lactosehaltige Permeat durch Ultrafiltration, insbesondere von Milch wie Magermilch, von Molke wie Sauer- oder Süßmolke oder eines Gemisches aus Sauer- und Süßmolke, eines Milchderivats oder eines Molkenderivats, erzeugt. Bevorzugt wird das lactosehaltige Permeat durch Ultrafiltration eines Milchpermeats, eines Permeats einer milchhaltigen Lösung wie einer Milchpulverlösung, eines Sauermolkenpermeats, eines Sauer- und Süßmolkenpermeats, eines Permeats einer sauermolkenhaltigen Lösung wie Sauermolkenpulverlösung, eines Süßmolkenpermeats, eines Permeats einer süßmolkenhaltigen Lösung wie einer Süßmolkenpulverlösung oder eines Permeats einer sauermolken- und süßmolkenhaltigen Lösung wie einer sauermolkenpulver- und süßmolkenpulverhaltigen Lösung erzeugt. Durch die Ultrafiltration ist mit besonderem Vorteil eine Abreicherung von in dem lactosehaltigen Substrat vorhandenen Proteinen erzielbar, welche ansonsten beim Durchführen von Schritt d) zu Störungen führen und sich insbesondere negativ auf die Sensorik des herzustellenden Produkts auswirken können.

Bevorzugt wird die Ultrafiltration unter Verwendung einer semipermeablen Polymermembran, welche eine Ausschlussgrenze (MWCO) von 10 kDa bis 20 kDa, bevorzugt von 10 kDa, aufweist, durchgeführt. Die semipermeable Polymermembran kann beispielsweise Polyethersulfon enthalten oder aus Polyethersulfon bestehen. Unter dem Ausdruck "Ausschlussgrenze" (Molecular Weight Cut-Off, MWCO) soll in diesem Zusammenhang die minimale Molekülmasse globulärer Moleküle verstanden werden, welche durch die für die Ultrafiltration verwendete semipermeable Polymermembran zu 90 % zurückgehalten werden.

In weiterer Ausgestaltung der Erfindung wird das lactosehaltige Substrat vor Durchführen von Schritt b) erhitzt und/oder sterilisiert. Dadurch können mit besonderem Vorteil Mikroorganismen, insbesondere pulverassoziierte Mikroorganismen im Falle der Verwendung eines pulverförmigen, lactosehaltigen Substrats oder einer Lösung eines solchen Substrats, abgetötet werden.

Beispielsweise kann das lactosehaltige Substrat vor Durchführen von Schritt b) insbesondere auf eine Temperatur von 60 °C bis 140 °C erhitzt werden. Weiterhin kann das lactosehaltige Substrat vor Durchführen von Schritt b) über einen Zeitraum von 1 s bis 5 min erhitzt werden.

Beispielsweise kann das lactosehaltige Substrat vor Durchführen von Schritt b) pasteurisiert, d.h. über einen Zeitraum von 15 s bis 35 s auf eine Temperatur von 72 °C bis 75 °C erhitzt werden. Beispielsweise kann das lactosehaltige Substrat vor Durchführen von Schritt b) über einen Zeitraum von 32 s auf eine Temperatur von 72 °C erhitzt werden.

Alternativ kann das lactosehaltige Substrat vor Durchführen von Schritt b) über einen Zeitraum von 1 s bis 3 s auf eine Temperatur von 120 °C erhitzt werden.

Alternativ kann das lactosehaltige Substrat vor Durchführen von Schritt b) während eines Zeitraums von 15 s bis 30 s auf 85 °C bis 134 °C erhitzt werden (sogenannte Hochpasteurisierung).

Alternativ kann das lactosehaltige Substrat vor Durchführen von Schritt b) über einen Zeitraum von 1 s bis 10 s auf 135 °C bis 150 °C erhitzt werden (sogenannte Ultrahocherhitzung).

Alternativ oder in Kombination kann das lactosehaltige Substrat vor Durchführen von Schritt b) durch Autoklavenerhitzung, insbesondere über einen Zeitraum von 20 min bis 40 min und bei einer Temperatur von 109 °C bis 120 °C sterilisiert werden.

Die im Rahmen der vorliegenden Erfindung verwendeten/verwendete β-Galactosidase und/oder β-Glucosidase katalysieren/katalysiert eine als Transgalactosylierung bezeichnete Reaktion, im Zuge derer Lactose in Glucose und einen Galactosylrest gespalten wird, wobei der Galactosylrest in Gegenwart von Fructose, insbesondere überschüssiger Fructose, auf Fructose unter Erzeugung von Lactulose übertragen wird.

Die Fructose wird dem lactosehaltigen Substrat vor Durchführen von Schritt b), d.h. vor der Behandlung mit der β-Galactosidase und/oder β-Glucosidase, zugegeben. Dabei kann die Fructose insbesondere in einem Anteil von 4 Gew.-% bis 30 Gew.-%, bevorzugt 5 Gew.-% bis 15 Gew.-%, besonders bevorzugt 8 Gew.-% bis 12 Gew.-%, dem lactosehaltigen Substrat zugegeben werden, bezogen auf das Gesamtgewicht eines Reaktionsgemisches, welches das lactosehaltige Substrat sowie die Fructose enthält. Die in diesem Absatz offenbarten Fructoseanteile haben sich als besonders vorteilhaft im Hinblick auf das Einstellen eines Fructoseüberschusses und mithin im Hinblick auf die Erzeugung von Lactulose herausgestellt.

Weiterhin kann die Fructose dem lactosehaltigen Substrat vor Durchführen von Schritt b) über einen Zeitraum von 10 min bis 6 h, insbesondere 15 min bis 3 h, bevorzugt 15 min bis 1 h, zugegeben werden.

Weiterhin kann die Fructose dem lactosehaltigen Substrat vor Durchführen von Schritt b) bei einer Temperatur von 5 °C bis 60 °C, insbesondere 5 °C bis 20 °C, bevorzugt 10 °C bis 15 °C, zugegeben werden.

In weiterer Ausgestaltung der Erfindung wird zum Durchführen von Schritt b) eine β-Galactosidase aus oder von *Aspergillus niger*, *Aspergillus oryzae*, *Kluyveromyces lactis*, *Bacillus licheniformis* oder *Bifidobacterium bifidum* verwendet. Die in diesem Absatz genannten β-Galactosidasen erlauben die Erzeugung von Lactulose unter besonders milden und insbesondere schonenden Reaktionsbedingungen.

Beispielsweise kann zum Durchführen von Schritt b) eine β-Galactosidase oder ein β-Galactosidasepräparat verwendet werden, welche bzw. welches aus oder von *Aspergillus niger* stammt und unter der Bezeichnung Maxilact kommerziell erhältlich ist.

Alternativ kann zum Durchführen von Schritt b) eine β-Galactosidase oder ein β-Galactosidasepräparat verwendet werden, welche bzw. welches aus oder von *Aspergillus* oryzae stammt und unter der Bezeichnung Enzeco® kommerziell erhältlich ist.

Alternativ kann zum Durchführen von Schritt b) eine β-Galactosidase oder ein β-Galactosidasepräparat verwendet werden, welche bzw. welches aus oder von *Kluyveromyces lactis* stammt und unter der Bezeichnung Lactozym, Biolactasa oder Ha-Lactase kommerziell erhältlich ist.

Alternativ kann zum Durchführen von Schritt b) eine β-Galactosidase oder ein β-Galactosidasepräparat verwendet werden, welche bzw. welches aus oder von *Bacillus licheniformis* stammt und unter der Bezeichnung Saphera kommerziell erhältlich ist.

Alternativ kann zum Durchführen von Schritt b) eine β-Galactosidase oder ein β-Galactosidasepräparat verwendet werden, welche bzw. welches aus oder von *Bifidobacterium bifidum* stammt und unter der Bezeichnung NOLA-Fit kommerziell erhältlich ist.

Die in den vorherigen Absätzen genannten β-Galactosidasen haben den Vorteil, dass es sich hierbei um bereits für Lebensmittel zugelassene Enzyme handelt.

Es versteht sich, dass zum Durchführen von Schritt b) auch eine Kombination von β-Galactosidasen, insbesondere eine Kombination von wenigstens zwei in den vorherigen Absätzen genannten β-Galactosidasen, verwendet werden kann.

In weiterer Ausgestaltung der Erfindung wird zum Durchführen von Schritt b) eine β-Glucosidase aus oder von *Aspergillus oryzae* oder *Pyrococcus furiosus* verwendet. Die in diesem Absatz genannten β-Glucosidasen erlauben die Erzeugung von Lactulose unter besonders milden und insbesondere schonenden Reaktionsbedingungen.

Es versteht sich, dass zum Durchführen von Schritt b) auch eine Kombination von β-Glucosidasen, insbesondere eine Kombination von wenigstens zwei im vorherigen Absatz genannten β-Glucosidasen, verwendet werden kann.

Weiterhin versteht sich, dass zum Durchführen von Schritt b) auch eine Kombination (wenigstens) einer β-Galactosidase und (wenigstens) einer β-Glucosidase verwendet werden kann. Bezüglich geeigneter β-Galactosidasen und β-Glucosidasen sei vollständig auf die vorherigen Absätze verwiesen.

Bevorzugt wird Schritt b) bei einer Temperatur von 2 °C bis 42 °C, insbesondere 5 °C bis 15 °C, vorzugsweise 5 °C bis 7 °C, durchgeführt. Insbesondere bevorzugt wird die Temperatur beim Durchführen von Schritt b) konstant gehalten. Auf diese Weise kann eine Beeinträchtigung der Enzymaktivität vermieden werden.

Weiterhin bevorzugt wird Schritt b) in einem pH-Bereich von 4,0 bis 7,0, insbesondere 4,0 bis 6,5, bevorzugt 4,5 bis 6,5, durchgeführt.

Weiterhin bevorzugt wird Schritt b) mit einer Enzymaktivität, insbesondere mit einer normierten Enzymaktivität, von 0,1 nkat bis 20 nkat, insbesondere 1 nkat bis 10 nkat, vorzugsweise 2 nkat, pro ml des lactosehaltigen Substrats durchgeführt.

Bevorzugt wird Schritt b) über einen Zeitraum von 12 h bis 72 h, insbesondere 24 h bis 60 h, vorzugsweise 40 h bis 56 h, durchgeführt.

Durch die in den vorangegangen Absätzen beschriebenen Verfahrensparameter des Schrittes b), welcher auch als Fermentationsschritt bezeichnet werden kann, sind mit besonderem Vorteil maximale Lactulosekonzentrationen realisierbar, ehe ein durch die β-Galactosidase und/oder β-Glucosidase katalysierter Abbau von Lactulose überwiegt. Dies gilt insbesondere in Bezug auf die offenbarten Zeiträume (Umsetzungszeiten). Insoweit hat sich überraschenderweise herausgestellt, dass ein breites Maximum der Lactulosekonzentration erreicht werden kann, ehe ein durch die β-Galactosidase und/oder β-Glucosidase katalysierter Abbau von Lactulose überwiegt. Dadurch besteht beispielsweise ausreichend Zeit, die β-Galactosidase und/oder die β-Glucosidase entweder in einem Batch oder nach Ausschleusen, insbesondere Abpumpen, aus einem Fermenter, in welchem der Schritt b) durchgeführt werden kann, durch Erhitzen zu inaktivieren.

In weiterer Ausgestaltung der Erfindung wird Schritt b)

- bei einer Temperatur von 2 °C bis 42 °C, insbesondere 5 °C bis 15 °C, bevorzugt 5 °C bis 7 °C,

- in einem pH-Bereich von 4,0 bis 7,0, insbesondere 4,0 bis 6,5, bevorzugt 4,5 bis 6,5,

- mit einer Enzymaktivität, insbesondere normierten Enzymaktivität, von 0,1 nkat bis 20 nkat, insbesondere 1 nkat bis 10 nkat, vorzugsweise 2 nkat, pro ml des lactosehaltigen Substrats und

- über einen Zeitraum von 12 h bis 72 h, insbesondere 24 h bis 60 h, vorzugsweise 40 h bis 56 h, durchgeführt.

Bezüglich der Vorteile dieser Ausgestaltung wird vollumfänglich auf die in den vorangegangenen Absätzen gemachten Ausführungen Bezug genommen. Die dort beschriebenen Vorteile gelten sinngemäß.

In weiterer Ausgestaltung der Erfindung wird das lactosehaltige Substrat durch Schritt a) in Form eines Süßmolkenpermeats oder in Form eines Permeats einer Lösung eines Süßmolkenderivats, insbesondere eines Süßmolkenpulvers, bereitgestellt und zum Durchführen von Schritt b) eine β-Galactosidase von *Kluyveromyces lactis,* beispielsweise eine β-Galactosidase oder ein β-Galactosidasepräparat, welche bzw. welches unter der Bezeichnung Lactozym, Biolactasa oder Ha-Lactase kommerziell erhältlich ist, verwendet.

Bevorzugt wird Schritt b) in diesem Fall

- bei einer Temperatur von 2 °C bis 42 °C, insbesondere 5 °C bis 15 °C, bevorzugt 5 °C bis 7 °C, und/oder

- in einem pH-Bereich von 5,0 bis 7,0, insbesondere 6,0 bis 7,0, bevorzugt 6,0 bis 6,5, und/oder

- mit einer Enzymaktivität, insbesondere mit einer normierten Enzymaktivität, von 0,1 nkat bis 20 nkat, insbesondere 1 nkat bis 10 nkat, vorzugsweise 2 nkat, pro ml des lactosehaltigen Substrats und/oder

- über einen Zeitraum von 12 h bis 72 h, insbesondere 24 h bis 60 h, bevorzugt 40 h bis 56 h, durchgeführt.

In weiterer Ausgestaltung der Erfindung wird das lactosehaltige Substrat durch Schritt a) in Form eines Sauermolkenpermeats oder in Form eines Permeats einer Lösung eines Sauermolkenderivats, insbesondere eines Sauermolkenpulvers, bereitgestellt und zum Durchführen von Schritt b) eine β-Galactosidase von *Aspergillus oryzae,* beispielsweise eine β-Galactosidase oder ein β-Galactosidasepräparat, welche bzw. welches unter der Bezeichnung Enzeco® kommerziell erhältlich ist, verwendet.

Bevorzugt wird Schritt b) in diesem Fall

- bei einer Temperatur von 2 °C bis 42 °C, insbesondere 5 °C bis 15 °C, bevorzugt 5 °C bis 7 °C, und/oder

- in einem pH-Bereich von 4,0 bis 6,0, insbesondere 4,0 bis 5,0, bevorzugt 4,5 bis 5,0, und/oder

- mit einer Enzymaktivität, insbesondere mit einer normierten Enzymaktivität, von 0,1 nkat bis 20 nkat, insbesondere 1 nkat bis 10 nkat, vorzugsweise 2 nkat, pro ml des lactosehaltigen Substrats und/oder

- über einen Zeitraum von 12 h bis 72 h, insbesondere 24 h bis 60 h, bevorzugt 40 h bis 56 h, durchgeführt.

In weiterer Ausgestaltung der Erfindung wird Schritt c) bei einer Temperatur von 90 °C bis 150 °C, insbesondere 100 °C bis 130 °C, bevorzugt 120 °C bis 130 °C, durchgeführt. Mit anderen Worten wird die β-Galactosidase und/oder β-Glucosidase gemäß weiterer Ausgestaltung der Erfindung durch Erhitzen auf eine Temperatur von 90 °C bis 150 °C, insbesondere 100 °C bis 130 °C, bevorzugt 120 °C bis 130 °C, inaktiviert. Die Inaktivierung der Enzyme/des Enzyms beruht in der Regel auf Denaturierungsprozessen. Durch eine Inaktivierung der β-Galactosidase und/oder β-Glucosidase wird, wie bereits erwähnt, mit besonderem Vorteil ein durch die β-Galactosidase und/oder β-Glucosidase katalysierter Lactuloseabbau vermieden und mithin die Voraussetzungen für eine nachfolgende Lactuloseanreicherung geschaffen.

Bevorzugt wird Schritt c) über einen Zeitraum von 1 s bis 360 s, insbesondere 1 s bis 20 s, vorzugsweise 8 s bis 16 s, durchgeführt.

Weiterhin kann nach Durchführen von Schritt c) unter Prozesskontrollgesichtspunkten der Lactuloseanteil in dem lactulosehaltigen Substrat bestimmt werden. Die Bestimmung des Lactuloseanteils kann beispielsweise mittels Hochleistungsflüssigkeitschromatographie (High Performance Liquid Chromatography, HPLC) erfolgen.

Das lactulosehaltige Substrat wird durch Nanofiltration und Diafiltration erzeugt. Die Diafiltration wird, insbesondere unmittelbar, nach der Nanofiltration durchgeführt. Durch eine Nano- und anschließende Diafiltration ist eine besonders wirkungsvolle Anreicherung von Lactulose unter gleichzeitiger Abreicherung, insbesondere Auswaschung, von Monosacchariden und/oder anderen Disacchariden (als Lactulose) erzielbar. Dadurch kann besonders wirkungsvoll eine übermäßige Süße, ein nachteiliger Einfluss auf den glykämischen Index sowie insbesondere ein unerwünscht hoher Energiegehalt des herzustellenden lactulosehaltigen Produkts vermieden werden.

Bevorzugt wird die Nanofiltration unter Verwendung einer semipermeablen Polymermembran durchgeführt, welche eine Ausschlussgrenze (MWCO) von 150 Da bis 750 Da, insbesondere 150 Da bis 300 Da, aufweist. Die semipermeable Membran kann beispielsweise ein aromatisches Polyamid (Aramid) enthalten oder aus einem solchen Polyamid bestehen. Unter dem Ausdruck "Ausschlussgrenze" (Molecular Weight Cut-Off, MWCO) soll in diesem Zusammenhang die minimale Molekülmasse globulärer Moleküle verstanden werden, welche durch die für die Nanofiltration verwendete semipermeable Polymermembran zu 90 % zurückgehalten werden. Eine Membran, wie in diesem Absatz beschrieben, ist beispielsweise unter der Bezeichnung TNF 3838 bei Toray Industries kommerziell erhältlich.

Weiterhin kann die Nanofiltration bei einer Temperatur von 5 °C bis 50 °C, insbesondere 40 °C bis 50 °C, bevorzugt 40 °C bis 45 °C, durchgeführt werden.

Weiterhin kann die Nanofiltration bei einem pH-Wert von 4,0 bis 7,0, insbesondere 5,0 bis 6,5, bevorzugt 6,0 bis 6,5, durchgeführt werden.

Weiterhin kann die Nanofiltration über einen Zeitraum von 1 h bis 12 h, insbesondere 2 h bis 8 h, bevorzugt 6 h bis 8 h, durchgeführt werden.

Bevorzugt wird die Dialfiltration unter Verwendung einer semipermeablen Polymermembran durchgeführt, welche eine Ausschlussgrenze (Molecular Weight Cut-Off, MWCO) von 150 Da bis 750 Da, insbesondere 150 Da bis 300 Da, aufweist. Die semipermeable Polymembran kann beispielsweise ein aromatisches Polyamid (Aramid) enthalten oder aus einem solchen Polyamid bestehen. Unter dem Ausdruck "Ausschlussgrenze" (Molecular Weight Cut-Off, MWCO) soll in diesem Zusammenhang die minimale Molekülmasse globulärer Moleküle verstanden werden, welche durch die für die Diafiltration verwendete semipermeable Polymermembran zu 90 % zurückgehalten werden.

Weiterhin kann die Diafiltration bei einer Temperatur von 5 °C bis 50 °C, insbesondere 40 °C bis 50 °C, bevorzugt 40 °C bis 45 °C, durchgeführt werden.

Weiterhin kann die Diafiltration bei einem pH-Wert von 4,0 bis 7,0, insbesondere 5,0 bis 6,5, bevorzugt 6,0 bis 6,5, durchgeführt werden.

Weiterhin kann die Diafiltration über einen Zeitraum von 1 h bis 12 h, insbesondere 2 h bis 8 h, bevorzugt 2 h bis 4 h, durchgeführt werden.

Die Nanofiltration wird bei einer transmembranen Druckdifferenz von 10 bar bis 50 bar, insbesondere 10 bar bis 40 bar, bevorzugt 20 bar bis 40 bar, durchgeführt. Dadurch lässt sich der Prozess besonders effizient gestalten, insbesondere hinsichtlich Energieverbrauch, Produktionszeit und Trennschärfe. Beispielsweise kann die Nanofiltration bei einer transmembranen Druckdifferenz von 30 bar durchgeführt werden. Hierdurch lässt sich in besonders vorteilhafter Weise ein Konzentrierungsfaktor i von 3 erreichen. Der Konzentrierungsfaktor ist über nachstehende Gleichung berechenbar:

$$i = \frac{V_F}{V_K} \, ,$$

wobei i Konzentrierungsfaktor, $V_F$ Feedvolumen und $V_K$ Konzentratvolumen bedeuten.

Unter dem Ausdruck "transmembrane Druckdifferenz $\Delta p_{TM}$" soll im Sinne der vorliegenden Erfindung der Mittelwert zwischen dem Druck am Moduleingang $p_1$ und am Modulausgang $p_2$ minus dem Druck auf der Permeatseite $p_3$ verstanden werden. Für organische Membranen gilt immer $p_3$ gleich Null. Die transmembrane Druckdifferenz ist über nachstehende Gleichung berechenbar:

$$\Delta p_{TM} = \frac{p_1 + p_2}{2} - p_3$$

Die transmembrane Druckdifferenz wird beim Durchführen von Schritt d) vor der Diafiltration vermindert, insbesondere von 30 bar auf 20 bar. Dadurch lassen sich unerwünschte Monosaccharide besonders effizient auswaschen, ohne dabei die Prozessdauer zu sehr zu erhöhen. Hierzu können beispielsweise pro zehn/hundert oder tausend Liter abgezogenes Permeat durch die gleiche Menge an destilliertem Wasser in einen Feedtank gegeben werden, bis ein Diafiltrationsfaktor D von 3 erreicht wird. Der Diafiltrationsfaktor D ist gemäß nachfolgender Gleichung berechenbar:

$$D = \frac{V_D}{V_K} ,$$

wobei D Diafiltrationsfaktor, $V_D$ Volumen des Diafiltrationsmediums und $V_K$ Konzentratvolumen bedeuten.

In weiterer Ausgestaltung der Erfindung wird die transmembrane Druckdifferenz während der Diafiltration, insbesondere diskontinuierlich oder stufenweise, reduziert, bevorzugt von 20 bar auf 12 bar. Diese Maßnahme fördert das Auswaschen von Monosacchariden. Die Reduzierung der transmembranen Druckdifferenz kann dabei diskontinuierlich, insbesondere stufenweise, oder kontinuierlich, insbesondere durch den entstehenden Flux geregelt, erfolgen. In weiterer Ausgestaltung der Erfindung weist das Verfahren ferner nachfolgenden Schritt e) auf:

e) Abfüllen des lactulosehaltigen Retentats in Verpackungseinheiten, insbesondere mit einem Füllvolumen von 100 ml bis 500 ml, bevorzugt 100 ml bis 250 ml, weiter bevorzugt 100 ml bis 150 ml, besonders bevorzugt 150 ml, oder mit einem Füllgewicht von 100 g bis 500 g , bevorzugt 100 g bis 250 g, weiter bevorzugt 100 g bis 150 g, besonders bevorzugt 150 g.

Weiterhin kann das lactulosehaltige Retentat, insbesondere vor Durchführen von Schritt e), gekühlt werden. Zum Beispiel kann das lactulosehaltige Retentat vor Durchführen von Schritt e) auf eine Temperatur von 2 °C bis 10 °C, insbesondere 4 °C bis 8 °C, bevorzugt 5 °C bis 7 °C, gekühlt werden. Vorzugsweise erfolgt das Kühlen des lactulosehaltigen Retentats unmittelbar nach Durchführen von Schritt d). Durch eine Kühlung des lactulosehaltigen Substrats kann mit besonderem Vorteil das Wachstum von Bakterien minimiert werden.

Weiterhin kann das lactulosehaltige Retentat, insbesondere vor Durchführen von Schritt e), angesäuert, d.h. mit einer Säure versetzt, werden. Beispielsweise kann das lactulosehaltige Retentat mit einer Säure ausgewählt aus der Gruppe bestehend aus Citronensäure, Äpfelsäure, Fumarsäure, Essigsäure, Ascorbinsäure, Bernsteinsäure, Phosphorsäure, Weinsäure, Milchsäure und Mischungen von wenigstens zwei der genannten Säuren versetzt werden. Vorzugsweise erfolgt das Ansäuern des lactulosehaltigen Retentats unmittelbar nach Durchführen von Schritt d). Durch ein Ansäuern des lactulosehaltigen Retentats lässt sich ebenfalls mit besonderem Vorteil das Wachstum von Bakterien minimieren. Außerdem modifiziert die Säure den Geschmack des lactulosehaltigen Retentats. Dadurch wird die Akzeptanz und Verzehrbereitschaft auf Seiten der Konsumenten gestärkt.

Weiterhin kann das lactulosehaltige Retentat, insbesondere vor Durchführen von Schritt e), aromatisiert, d.h. mit einem Aromastoff versehen, werden. Beispielsweise kann das lactulosehaltige Retentat mit einem Aromastoff versetzt werden, welcher ausgewählt ist aus der Gruppe bestehend aus 4-Hydroxy-2,5-dimethyl-3(2H)-furanon, 2-Methylbuttersäureethylester, (R)-γ-Decalacton, δ-Decalacton, Terpineol, Geraniol, Linalool, 2-Methylbuttersäure, Essigsäureamylester, 2-Heptanol, 2-Heptanon, Anthranilsäuremethylester, Hexansäureethylester, Ethyl-methylphenylglycidat, Thioterpineol, (Z)-3-Hexenal, (+)-Nootkaton, Himbeerketon, Benzaldehyd, Hexanal, Phenylacetaldehyd, Eugenol, 3-Methyl-2-butanon, 3-Hydroxy-2-butanon, (E)-2-Hexenal, 3-Hydroxybuttersäureethylester, Phenylethylalkohol, α-Terpineol, Thymol, (Z)-6-Nonenal, 2,6-Dimethyl-5-heptenal, (R)-Limonen, Acetaldehyd, Decanal, trans-4,5-Epoxy-(E)-2-decenal, Essigsäurenerylester, Essigsäureoctylester, 2-Methyl-4-propyl-1,3-oxathian, β-Ionon, Hexansäureethylester, Buttersäurehexylester, Hexansäurehexylester, Zimtsäuremethylester, (E)-/(Z)-Marmelolacton, α-Pharnesen, Hexanol, 3-Hydroxy-β-ionol, 5,6-Dihydroxy-β-ionon, 4-Methoxy-2-methyl-2-butanthiol, Citral (Isomerengemisch aus Geranial und Neral), Myrcen und Mischungen von wenigstens zwei der genannten Aromastoffe. Durch einen Aromatisierungsschritt kann dem herzustellenden lactulosehaltigen Produkt mit besonderem Vorteil ein Geschmack verliehen werden, welcher bei den Konsumenten zu einer erhöhten Akzeptanz und mithin zu einer erhöhten Verzehrbereitschaft führt. Bevorzugt erfolgt ein Aromatisieren des lactulosehaltigen Retentats unmittelbar nach Durchführen von Schritt d).

Weiterhin kann es bevorzugt sein, wenn das lactulosehaltige Retentat, insbesondere vor Durchführen von Schritt e), gekühlt und/oder angesäuert und/oder aromatisiert wird, besonders bevorzugt unmittelbar nach Durchführen von Schritt d). Besonders bevorzugt wird das lactulosehaltige Retentat vor Durchführen von Schritt e) gekühlt, angesäuert und aromatisiert, insbesondere unmittelbar nach Durchführen von Schritt d). Bezüglich der Vorteile der in diesem Absatz beschriebenen Verfahrensmaßnahmen wird vollumfänglich auf die in den vorherigen Absätzen beschriebenen Vorteile Bezug genommen. Die dort insoweit beschriebenen Vorteile gelten sinngemäß.

Weiterhin kann das lactulosehaltige Retentat, insbesondere vor Durchführen von Schritt e), nochmals erhitzt werden. Beispielsweise kann das lactulosehaltige Retentat vorzugsweise vor Durchführen von Schritt e) nochmals auf eine Temperatur von 70 °C bis 150 °C, insbesondere 90 °C bis 150 °C, bevorzugt 135 °C bis 150 °C, erhitzt werden. Bevorzugt wird das lactulosehaltige Retentat hierzu über einen Zeitraum von 1 s bis 120 s, insbesondere 1 s bis 60 s, bevorzugt 1 s bis 10 s, erhitzt. Beispielsweise kann das lactulosehaltige Substrat einer Ultrahocherhitzung unterworfen werden, d.h. über einen Zeitraum von 1 s bis 10 s auf eine Temperatur von 135 °C bis 150 °C, insbesondere über einen Zeitraum von 8 s auf 145 °C, erhitzt werden. Durch die in diesem Absatz beschriebenen Verfahrensmaßnahmen lässt sich die Haltbarkeit des herzustellenden lactulosehaltigen Produkts mit besonderem Vorteil signifikant erhöhen, insbesondere außerhalb einer Kühlkette. Beispielsweise ist es mittels der in diesem Absatz beschriebenen Verfahrensmaßnahmen möglich, eine Haltbarkeit des herzustellenden lactulosehaltigen Produkts von mindestens sechs Wochen bei einer Lagerungstemperatur von 6 °C zu erreichen. Bevorzugt erfolgt ein nochmaliges Erhitzen des lactulosehaltigen Retentats nach einem Kühlungs- und/oder Ansäuerungs- und/oder Aromatisierungsschritt, insbesondere wie in den vorangegangenen Absätzen beschrieben. Weiterhin kann das lactulosehaltige Retentat carbonisiert, d. h. mit Kohlendioxid versetzt, werden. Dadurch kann mit besonderem Vorteil beim Konsumenten ein prickelnder oder erfrischender Effekt hervorgerufen werden.

Grundsätzlich ist es bevorzugt, wenn das lactulosehaltige Produkt in Form eines lactulosehaltigen Retentats, insbesondere wie in den vorangegangenen Absätzen beschrieben, hergestellt wird. Dementsprechend kann das lactulosehaltige Produkt insbesondere als Milch- oder Molkenretentat, insbesondere als Süß- oder Sauermolkenretentat, hergestellt werden. Alternativ kann das lactulosehaltige Produkt als Retentat einer Milchpulverlösung, einer Süßmolkenpulverlösung oder einer Sauermolkenpulverlösung hergestellt werden.

Alternativ kann das lactulosehaltige Retentat, insbesondere vor Durchführen von Schritt e), unter Erzeugung eines lactulosehaltigen Konzentrats (sogenanntes "Lactulosekonzentrat"), insbesondere lactulosehaltigen Sirups (sogenannter "Lactosesirup"), konzentriert werden. Hierzu kann das lactulosehaltige Retentat beispielsweise eingedampft und/oder einer Umkehrosmose unterworfen werden. Mit anderen Worten kann das lactulosehaltige Produkt alternativ in Form eines lactulosehaltigen Konzentrats, insbesondere in Form eines lactulosehaltigen Sirups, hergestellt werden.

Weiterhin kann das lactulosehaltige Retentat, insbesondere vor Durchführen von Schritt e), in einen Feststoff, insbesondere in ein Pulver oder Granulat, überführt werden. Hierzu kann das lactulosehaltige Retentat beispielsweise einer Lyophilisation oder Sprühtrocknung unterworfen werden. Mit anderen Worten kann das lactulosehaltige Produkt alternativ in Form eines Feststoffes, insbesondere in Form eines Pulvers oder Granulats, hergestellt werden.

Gemäß einem zweiten Aspekt betrifft die Erfindung ein lactulosehaltiges Produkt.

Das lactulosehaltige Produkt weist eine Lactulosekonzentration (genauer gesagt, eine Lactulosemassenkonzentration), insbesondere bei einer Temperatur von 20 °C bis 25 °C, von 10 g/l bis 50 g/l, insbesondere 20 g/l bis 40 g/l, bevorzugt 20 g/l bis 30 g/l, auf. Durch die in diesem Absatz beschriebenen Lactulosekonzentrationen können besonders wirkungsvolle Lactulose-Dosen verfügbar gemacht werden, wodurch sich das lactulosehaltiges Produkt insbesondere für die Ernährung von älteren und/oder pflegebedürftigen und/oder rehabilitationbedürftigen Personen, insbesondere mit Verdauungsstörungen, verwenden lässt.

Weiterhin weist das lactulosehaltige Produkt eine Lactosekonzentration, (genauer gesagt, eine Lactosemassenkonzentration), insbesondere bei einer Temperatur von 20 °C bis 25 °C, von 10 g/l bis 100 g/l, insbesondere 20 g/l bis 90 g/l, bevorzugt 20 g/l bis 30 g/l, auf. Da es sich bei Lactose um ein natürliches Präbiotikum handelt, wird der Effekt des lactulosehaltigen Produkts verstärkt. Durch die geringe Süßkraft lässt sich weiterhin eine übermäßige Süße vermeiden.

Weiterhin ist es bevorzugt, wenn das lactulosehaltige Produkt eine Glucosekonzentration, (genauer gesagt, eine Glucosemassenkonzentration), insbesondere bei einer Temperatur von 20 °C bis 25 °C, $\leq$ 15 g/l, insbesondere von 5 g/l bis 15 g/l, bevorzugt 5 g/l bis 10 g/l, aufweist. Dadurch lässt sich mit besonderem Vorteil eine übermäßige Süße, und insbesondere ein nachteiliger Einfluss des lactulosehaltigen Produkts auf den glykämischen Index vermeiden.

Weiterhin ist es bevorzugt, wenn das lactulosehaltige Produkt eine Galactosekonzentration, (genauer gesagt, eine Galactosemassenkonzentration), insbesondere bei einer Temperatur von 20 °C bis 25 °C, $\leq$ 15 g/l, insbesondere 5 g/l bis 15 g/l, bevorzugt 5 g/l bis 10 g/l, aufweist. Dadurch lässt sich mit besonderem Vorteil ebenfalls eine übermäßige Süße sowie insbesondere ein nachteiliger Einfluss des lactulosehaltiges Produkts auf den glykämischen Index vermeiden.

Weiterhin weist das lactulosehaltige Produkt eine Fructosekonzentration, (genauer gesagt, eine Fructosemassenkonzentration), insbesondere bei einer Temperatur von 20 °C bis 25 °C, < 45 g/l, insbesondere 10 g/l bis 30 g/l, bevorzugt 20 g/l bis 30 g/l, auf. Dadurch kann mit besonderem Vorteil ebenfalls einer übermäßigen Süße sowie insbesondere einem nachteiligen Einfluss des lactulosehaltiges Produkts auf den glykämischen Index vorgebeugt werden.

Bei dem lactulosehaltigen Produkt kann es sich grundsätzlich um ein Lebensmittelprodukt, insbesondere um ein Getränk, oder um ein pharmazeutisches Produkt, insbesondere in Form eines lactulosehaltigen Konzentrats, handeln. Bevorzugt handelt es sich bei dem lactulosehaltigen Produkt um ein präbiotisches Lebensmittelprodukt, insbesondere um ein präbiotisches Getränk. Bei dem Konzentrat kann es sich insbesondere um einen Sirup, d.h. um eine dickflüssige, konzentrierte Lösung, handeln.

Bevorzugt handelt es sich bei dem lactulosehaltigen Produkt um ein präbiotisches Lebensmittelprodukt, insbesondere um ein präbiotisches Milch- oder Molkenprodukt, bevorzugt um ein präbiotisches Milch- oder Molkengetränk. Bei dem Molkenprodukt kann es sich um ein Sauermolkenprodukt, Süßmolkenprodukt oder um ein Molkenmischprodukt, d. h. um ein aus Sauermolke und Süßmolke hergestelltes Molkenprodukt, handeln. Bei dem Molkengetränk kann es sich insbesondere um ein Sauermolkengetränk, Süßmolkengetränk oder um ein Molkenmischgetränk, d.h. um ein aus Sauer- und Süßmolke hergestelltes Getränk, handeln.

Das lactulosehaltige Produkt liegt vorzugsweise in Form eines lactulosehaltigen Retentats, insbesondere wie in den vorangegangenen Absätzen beschrieben, vor. Dementsprechend kann das lactulosehaltige Produkt insbesondere als Milch- oder Molkenretentat, insbesondere als Sauermolkenretentat, Süßmolkenretentat oder Sauer- und Süßmolkenretentat, vorliegen. Weiterhin kann das lactulosehaltige Produkt als Retentat einer milchhaltigen Lösung wie einer Milchpulverlösung, einer sauermolkenhaltigen Lösung wie einer sauermolkenpulverhaltigen Lösung, einer süßmolkenhaltigen Lösung wie einer süßmolkenpulverhaltigen Lösung oder einer sauermolken- und süßmolkenhaltigen Lösung wie einer sauermolkenpulver- und süßmolkenpulverhaltigen Lösung vorliegen.

Weiterhin kann das lactulosehaltige Produkt als Feststoff, insbesondere als Pulver oder Granulat, vorliegen.

Weiterhin kann das lactulosehaltige Produkt allgemein in Form eines lactulosehaltigen Konzentrats, insbesondere lactulosehaltigen Sirups, vorliegen.

Weitere Merkmale und Vorteile der Erfindung ergeben sich aus der nachfolgenden Beschreibung von Ausführungsbeispielen. Dabei können einzelne Merkmale jeweils für sich alleine oder in Kombination miteinander verwirklicht sein. Die Ausführungsbeispiele dienen lediglich einer weiteren Erläuterung der Erfindung, ohne dass diese hierauf beschränkt sein soll.

FIGURENKURZBESCHREIBUNG

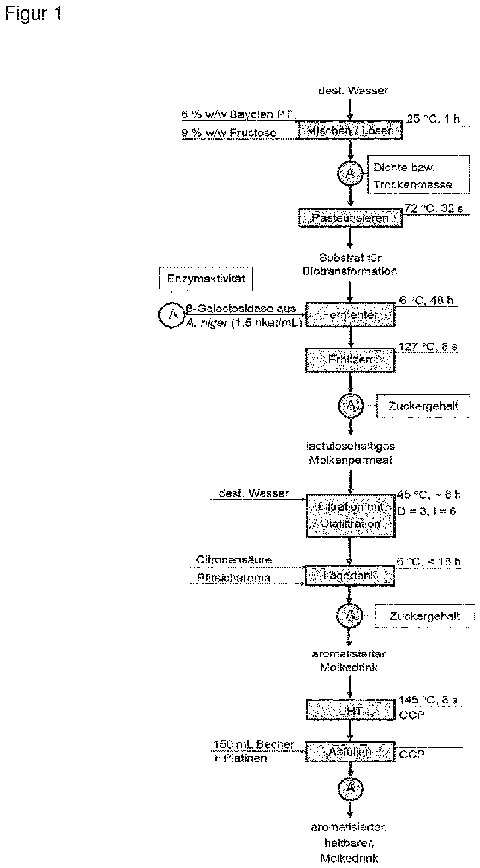

Figur 1 zeigt schematisch ein Flussdiagramm zur Herstellung eines erfindungsgemäßen lactulosehaltigen Produkts (wobei A Analyse zur Prozesskontrolle und CCP kritischer Kontrollpunkt bedeuten).

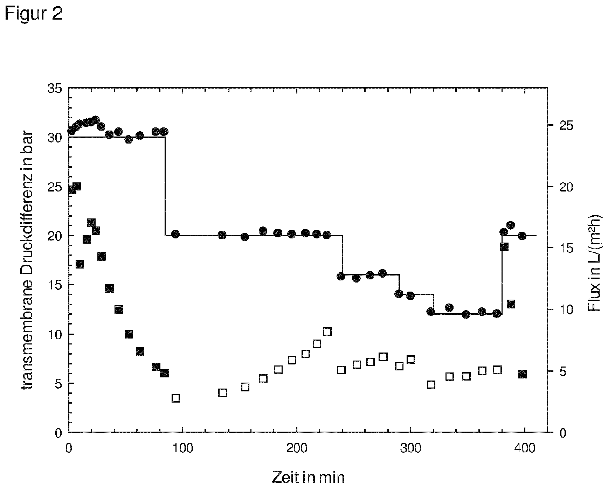

Die Figur 2 zeigt grafisch den theoretischen Druckgradient (durchgezogene Linie), die gemessenen transmembranen Druckdifferenzen (Kreise) sowie den Flux (Quadrate, offen: Diafiltration; Quadrate, geschlossen: Nanofiltration) während der Nano- und Diafiltration zur Herstellung eines präbiotischen Getränks.

BEISPIELTEIL

1. Ausführungsbeispiel zur Herstellung eines OneShot-Lactulosedrinks

Prozessplan zur Herstellung eines OneShot (150 mL) Molkedrinks mit einer präbiotisch wirksamen Dosis enzymatisch generierter Lactulose

1.1. Bereitstellen des Substrats für die Biotransformation

Für die Biotransformation wurde eine Lösung hergestellt, die 6 % Bayolan PT und 9 % Fructose (kristallin, Hamburg Fructose GmbH, Deutschland) enthielt (Schritt a). Diese wurde für 1 h bei Raumtemperatur gerührt. Anschließend wurde die Lösung pasteurisiert (72 °C, 32 s), um den pulverassoziierten Keimgehalt zu reduzieren. Danach wurde das Substrat für die Biotransformation in einen Fermenter überführt.

1.2. Biotransformation

Das Substrat für die Biotransformation wurde im Fermenter auf 6,0 $\pm$ 0,2 °C gekühlt. Anschließend wurde β-Galactosidase aus *Aspergillus niger* mit normierter Enzymaktivität (1,5 nkat/mL Substrat) zugegeben. Damit wurde die Biotransformation gestartet (Schritt b). Während der Biotransformation war darauf zu achten, dass Temperaturschwankungen (max. $\pm$ 0,2 °C) vermieden werden, da sonst die Enzymaktivität beeinflusst wird. Nach 48 h wurde das Enzym hitzeinaktiviert (Schritt c) (127 °C, 8 s) und der Zuckergehalt mittels HPLC-ELSD kontrolliert.

1.3. Nano- und Diafiltration

Für die Nano- und Diafiltration (Schritt d) wurde ein eigens entwickelter Prozess zur Fraktionierung von Mono- und Disacchariden verwendet. Dabei wurde das lactulosehaltige Molkenpermeat insgesamt sechsfach konzentriert und dreifach diafiltriert. Konkret wurde die Nano- und Diafiltration nach folgendem Prozessplan durgeführt: Für die Nanofiltration zum Herstellen des prebiotischen Getränks wurde die Membran TNF 3838 von Toray Industries verwendet. Die aktive Schicht der Membran bestand aus aromatischen Polyamiden und hatte eine nominelle Trenngrenze von 150 bis 300 Da.

Die Filtration wurde bei 45 °C durchgeführt, während die transmembrane Druckdifferenz $\Delta p_{TM}$ variiert wurde.

Während dem ersten Schritt des Konzentrierens betrug $\Delta p_{TM}$=30 bar. Sobald ein Konzentrierungsfaktor i = 3 (Gl. 1) erreicht wurde, wurde der Druck auf 20 bar eingestellt und die Diafiltration eingeleitet. Dazu wurden pro 10 L abgezogenes Permeat, 10 L destilliertes Wasser in den Feedtank gegeben, bis ein Diafiltrationsfaktor D = 3 (Gl. 2) erreicht wurde. Während der Diafiltration wurde der Druck stufenweise auf 12 bar herunterreguliert. Ziel des Druckgradienten war es, den Flux J $\leq$ 10 L/(m²·h) zu halten. Anschließend wurde erneut konzentriert bis ein Endkonzentrierungsfaktor von i = 6 erreicht wurde. Der Druck wurde dazu auf 20 bar eingestellt.

$$i = \frac{V_F}{V_K} \tag{1}$$

$$D = \frac{V_D}{V_K} \tag{2}$$

mit: $V_F$: Feedvolumen, $V_K$: Konzentratvolumen und $V_D$: Volumen des Diafiltrationsmediums

Tab. 1: Prozessplan (Nanofiltration) zum Herstellen eines präbiotischen Getränks bei 45 °C mit einem Feedvolumen von 180 L.

|  | Zugabe Wasser (á 10 L) | Abzug Permeat | Druck | Verbleibendes Volumen |
|---|---|---|---|---|
| NF I | - | 120 L | 30 bar | 60 L |
| DF | 90 L | 90 L | 20 bar | 60 L |
|  | 40 L | 40 L | 16 bar | 60 L |
|  | 20 L | 20 L | 14 bar | 60 L |
|  | 30 L | 30 L | 12 bar | 60 L |
| NF II | - | 30 L | 20 bar | 30 L |

Das Retentat wurde anschließend mit Citronensäure gesäuert und aromatisiert (Pfirsicharoma 321937, Symrise AG, Deutschland). Das Produkt wurde in einen Lagertank überführt und ist bei $\leq$ 6 °C bis zur weiteren Verarbeitung (< 18 h) lagerbar.

1.4. Erhitzen und Abfüllen

Der aromatisierte Molkedrink wurde abschließend erhitzt (145 °C, 8s) und in 150 mL Becher abgefüllt (Schritt e). Die Becher wurden versiegelt und sofort kühl gelagert.

Der Molkedrink wies die in nachfolgender Tabelle 2 wiedergegebene Zusammensetzung auf.

Tabelle 2: Zusammensetzung des OneShot Lactulosedrinks (Membran: Toray TNF 3838; $\vartheta$ = 45 °C)

| | |
|---|---|
| Fructose in g/L | 28 |
| Lactulose in g/L | 16 |
| Lactose in g/L | 92 |
| Trockenmasse in g/L | 195 |
| pH-Wert | 4,5 |
| Proteingehalt in g/L | 5 |

**Patentansprüche**

1. Verfahren zum Herstellen eines lactulosehaltigen Produkts, aufweisend die folgenden Schritte:

a) Bereitstellen eines lactosehaltigen Substrats in Form eines lactosehaltigen Permeats, wobei es sich bei dem lactosehaltigen Permeat um ein Permeat von Milch, von Molke oder eines Milch- oder Molkenderivats handelt,
b) Behandeln des lactosehaltigen Substrats mit einer β-Galactosidase und/oder einer β-Glucosidase in Gegenwart von Fructose unter Erzeugung eines lactulosehaltigen Substrats, wobei die Fructose dem lactosehaltigen Substrat vor Durchführen von Schritt b) zugegeben wird,
c) Inaktivieren der β-Galactosidase und/oder der β-Glucosidase durch Erhitzen und
d) Erzeugen eines lactulosehaltigen Retentats durch Nanofiltration und Diafiltration des lactulosehaltigen Substrats, wobei die Diafiltration nach der Nanofiltration durchgeführt, die Nanofiltration bei einer transmembranen Druckdifferenz von 10 bar bis 50 bar durchgeführt und die transmembrane Druckdifferenz vor dem Diafiltrieren vermindert wird.

2. Verfahren nach Anspruch 1, **dadurch gekennzeichnet, dass** das lactosehaltige Permeat durch Ultrafiltration von Milch, von Molke, eines Milchderivats oder eines Molkenderivats erzeugt wird.

3. Verfahren nach Anspruch 1 oder 2, **dadurch gekennzeichnet, dass** das lactosehaltige Substrat vor Durchführen von Schritt b) erhitzt, insbesondere pasteurisiert, und/oder sterilisiert wird.

4. Verfahren nach einem der vorhergehenden Ansprüche, **dadurch gekennzeichnet, dass** zum Durchführen von Schritt b) eine β-Galactosidase von *Aspergillus niger*, *Aspergillus oryzae*, *Kluyveromyces lactis*, *Bacillus licheniformis* oder *Bifidobacterium bifidum* und/oder eine β-Glucosidase von *Aspergillus oryzae* oder *Pyrococcus furiosus* verwendet wird.

5. Verfahren nach einem der vorhergehenden Ansprüche, **dadurch gekennzeichnet, dass** Schritt b)

   - bei einer Temperatur von 2 °C bis 42 °C, insbesondere 5 °C bis 15 °C, bevorzugt 5 °C bis 7 °C,
   - in einem pH-Bereich von 4,0 bis 7,0, insbesondere 4,0 bis 6,5, bevorzugt 4,5 bis 6,5,
   - mit einer Enzymaktivität, insbesondere normierten Enzymaktivität, von 0,1 nkat bis 20 nkat, insbesondere 1 nkat bis 10 nkat, vorzugsweise 2 nkat, pro ml des lactosehaltigen Substrats und
   - über einen Zeitraum von 12 h bis 72 h, insbesondere 24 h bis 60 h, bevorzugt 40 h bis 56 h,

   durchgeführt wird.

6. Verfahren nach einem der vorhergehenden Ansprüche, **dadurch gekennzeichnet, dass** das lactosehaltige Substrat durch Schritt a) in Form eines Süßmolkenpermeats oder Permeats einer Lösung eines Süßmolkenderivats bereitgestellt und zum Durchführen von Schritt b) eine β-Galactosidase von *Kluyveromyces lactis* verwendet wird.

7. Verfahren nach einem der Ansprüche 1 bis 5, **dadurch gekennzeichnet, dass** das lactosehaltige Substrat durch Schritt a) in Form eines Sauermolkenpermeats oder Permeats einer Lösung eines Sauermolkenderivats bereitgestellt und zum Durchführen von Schritt b) eine β-Galactosidase von *Aspergillus oryzae* verwendet wird.

8. Verfahren nach einem der vorhergehenden Ansprüche, **dadurch gekennzeichnet, dass** Schritt c) bei einer Temperatur von 90 °C bis 150 °C, insbesondere 100 °C bis 130 °C, bevorzugt 120 °C bis 130 °C, durchgeführt wird.

9. Verfahren nach einem der vorhergehenden Ansprüche, **dadurch gekennzeichnet, dass** die Nanofiltration bei einer transmembranen Druckdifferenz von 10 bar bis 40 bar, bevorzugt 20 bar bis 40 bar, durchgeführt wird.

10. Verfahren nach einem der vorhergehenden Ansprüche, **dadurch gekennzeichnet, dass** die transmembrane Druckdifferenz vor dem Diafiltrieren von 30 bar auf 20 bar vermindert wird.

11. Verfahren nach einem der vorhergehenden Ansprüche, **dadurch gekennzeichnet, dass** die transmembrane Druckdifferenz während der Dialfiltration stufenweise reduziert wird, insbesondere von 20 bar auf 12 bar.

12. Verfahren nach einem der vorhergehenden Ansprüche, **dadurch gekennzeichnet, dass** das Verfahren ferner nachfolgenden Schritt e) aufweist:
e) Abfüllen des lactulosehaltigen Retentats in Verpackungseinheiten, insbesondere mit einem Füllvolumen von 100 ml bis 500 ml, bevorzugt 100 ml bis 250 ml, weiter bevorzugt 100 ml bis 150 ml, besonders bevorzugt 150 ml.

13. Lactulosehaltiges Produkt herstellbar nach einem Verfahren gemäß der Ansprüche 1-12, aufweisend

- eine Lactulosekonzentration von 10 g/L bis 50 g/L,
- eine Lactosekonzentration von 10 g/L bis 100 g/L und
- eine Fructosekonzentration < 45 g/L.

**Claims**

1. A method for producing a lactulose-containing product, comprising the following steps:

    a) providing a lactose-containing substrate in the form of a lactose-containing permeate, wherein the lactose-containing permeate is a permeate of milk, whey or of a milk or whey derivative,
    b) treating the lactose-containing substrate with a β-galactosidase and/or a β-glucosidase in the presence of fructose, thereby generating a lactulose-containing substrate, wherein the fructose is admixed with the lactose-containing substrate before carrying out step b),
    c) inactivating the β-galactosidase and/or the β-glucosidase by heating, and
    d) generating a lactulose-containing retentate by nanofiltration and diafiltration of the lactulose-containing substrate, wherein the diafiltration is carried out after the nanofiltration, the nanofiltration is carried out at a transmembrane pressure difference of 10 bar to 50 bar, and the transmembrane pressure difference is reduced before diafiltration.

2. The method according to claim 1, **characterized in that** the lactose-containing permeate is generated by the ultrafiltration of milk, whey, a milk derivative or a whey derivative.

3. The method according to claim 1 or claim 2, **characterized in that** the lactose-containing substrate is heated, in particular pasteurized, and/or sterilized before carrying out step b).

4. The method according to any of the preceding claims, **characterized in that** a β-galactosidase of *Aspergillus niger*, *Aspergillus oryzae*, *Kluyveromyces lactis*, *Bacillus licheniformis* or *Bifidobacterium bifidum* and/or a β-glucosidase of *Aspergillus oryzae* or *Pyrococcus furiosus* is used to carry out step b).

5. The method according to any of the preceding claims, **characterized in that** step b) is carried out

    - at a temperature of 2°C to 42°C, in particular 5°C to 15°C, preferably 5°C to 7°C,
    - in a pH range of 4.0 to 7.0, in particular 4.0 to 6.5, preferably 4.5 to 6.5,
    - with an enzyme activity, in particular a standardized enzyme activity, of 0.1 nkat to 20 nkat, in particular 1 nkat to 10 nkat, preferably 2 nkat, per ml of the lactose-containing substrate, and
    - over a time period of 12 h to 72 h, in particular 24 h to 60 h, preferably 40 h to 56 h.

6. The method according to any of the preceding claims, **characterized in that** the lactose-containing substrate is provided by step a) in the form of a sweet whey permeate or a permeate of a solution of a sweet whey derivative, and a β-galactosidase of *Kluyveromyces lactis* is used to carry out step b).

7. The method according to any of claims 1 to 5, **characterized in that** the lactose-containing substrate is provided by step a) in the form of an acid whey permeate or a permeate of a solution of an acid whey derivative, and a β-galactosidase of *Aspergillus oryzae* is used to carry out step b).

8. The method according to any of the preceding claims, **characterized in that** step c) is carried out at a temperature of 90°C to 150°C, in particular 100°C to 130°C, preferably 120°C to 130°C.

9. The method according to any of the preceding claims, **characterized in that** the nanofiltration is carried out at a transmembrane pressure difference of 10 bar to 40 bar, preferably 20 bar to 40 bar.

10. The method according to any of the preceding claims, **characterized in that** the transmembrane pressure difference is reduced from 30 bar to 20 bar before diafiltration.

11. The method according to any of the preceding claims, **characterized in that** the transmembrane pressure difference is reduced gradually during the diafiltration, in particular from 20 bar to 12 bar.

**12.** The method according to any of the preceding claims, **characterized in that** the method further comprises the following step e):
e) packaging the lactulose-containing retentate in packaging units, in particular to a filling volume of 100 ml to 500 ml, preferably 100 ml to 250 ml, further preferably 100 ml to 150 ml, particularly preferably 150 ml.

**13.** A lactulose-containing product that can be produced in accordance with a method according to claims 1-12, having

- a lactulose concentration of 10 g/L to 50 g/L,
- a lactose concentration of 10 g/L to 100 g/L and
- a fructose concentration of < 45 g/L.

**Revendications**

**1.** Procédé de fabrication d'un produit contenant du lactulose, présentant les étapes suivantes :

a) Fourniture d'un substrat contenant du lactose sous forme d'un perméat contenant du lactose, sachant qu'il s'agit pour ce perméat contenant du lactose d'un perméat de lait, de lactosérum ou d'un dérivé du lait ou du lactosérum,
b) Traitement du substrat contenant du lactose avec un β-galactosidase et/ou un β-glucosidase en présence de fructose pour former un substrat contenant du lactulose, sachant que le fructose est ajouté au substrat contenant du lactose avant l'exécution de l'étape b),
c) Inactivation du β-galactosidase et/ou du β-glucosidase par chauffe et
d) Création d'un rétentat contenant du lactulose par nanofiltration et diafiltration du substrat contenant du lactulose, sachant que la diafiltration est exécutée après la nanofiltration, que la nanofiltration est exécutée à une pression différentielle transmembranaire de 10 à 50 bar et que la pression différentielle transmembranaire est réduite avant la diafiltration.

**2.** Procédé selon la revendication 1, **caractérisé en ce que** le perméat contenant du lactose est créé par ultrafiltration du lait, du lactosérum, d'un dérivé du lait ou d'un dérivé du lactosérum.

**3.** Procédé selon la revendication 1 ou 2, **caractérisé en ce que** le substrat contenant du lactose est chauffé avant l'exécution de l'étape b), notamment pasteurisé, et/ou stérilisé.

**4.** Procédé selon l'une des revendications précédentes, **caractérisé en ce que** pour exécuter l'étape b), un β-galactosidase *d'Aspergillus niger*, *Aspergillus oryzae*, *Kluyveromyces lactis*, *Bacillus licheniformis* ou de *Bifidobacterium bifidum* et/ou un β-glucosidase *d'Aspergillus oryzae* ou de *Pyrococcus furiosus* est utilisé.

**5.** Procédé selon l'une des revendications précédentes, **caractérisé en ce que** l'étape b) est exécutée

- à une température de 2 °C à 42 °C, notamment 5 °C à 15 °C, de préférence 5 °C à 7 °C,
- dans une plage de pH de 4,0 à 7,0, notamment de 4,0 à 6,5, de préférence de 4,5 à 6,5,
- avec une activité enzymatique, notamment activité enzymatique normalisée, de 0,1 nkat à 20 nkat, notamment de 1 nkat à 10 nkat, de préférence 2 nkat, par ml de substrat contenant du lactose et
- dans une période de 12 h à 72 h, notamment de 24 h à 60 h, de préférence de 40 h à 56 h.

**6.** Procédé selon l'une des revendications précédentes, **caractérisé en ce que** le substrat contenant du lactose est fourni par l'étape a) sous forme de perméat de lactosérum doux ou de perméat d'une solution d'un dérivé du lactosérum doux et qu'un β-galactosidase de *Kluyveromyces lactis* est utilisé pour exécuter l'étape b).

**7.** Procédé selon l'une des revendications 1 à 5, **caractérisé en ce que** le substrat contenant du lactose est fourni par l'étape a) sous forme de perméat de lactosérum acide ou de perméat d'une solution d'un dérivé du lactosérum acide et qu'un β-galactosidase *d'Aspergillus oryzae* est utilisé pour exécuter l'étape b).

**8.** Procédé selon l'une des revendications précédentes, **caractérisé en ce que** l'étape c) est exécutée à une température comprise entre 90 °C et 150 °C, notamment entre 100 °C et 130 °C, de préférence entre 120 °C et 130 °C.

**9.** Procédé selon l'une des revendications précédentes, **caractérisé en ce que** la nanofiltration est exécutée à une pression différentielle transmembranaire comprise entre 10 bar et 40 bar, de préférence entre 20 bar et 40 bar.

10. Procédé selon l'une des revendications précédentes, **caractérisé en ce que** la pression différentielle transmembranaire est réduite de 30 bar à 20 bar avant la diafiltration.

11. Procédé selon l'une des revendications précédentes, **caractérisé en ce que** la pression différentielle transmembranaire est réduite progressivement pendant la diafiltration, notamment de 20 bar à 12 bar.

12. Procédé selon l'une des revendications précédentes, **caractérisé en ce que** le procédé présente, de plus, l'étape e) suivante :
    e) Transvasement du rétentat contenant du lactulose dans des unités de conditionnement, notamment avec un volume de remplissage compris entre 100 ml et 500 ml, de préférence entre 100 ml et 250 ml, mais encore de préférence entre 100 ml et 150 ml, en particulier 150 ml.

13. Produit contenant du lactulose, fabricable selon un procédé conformément aux revendications 1 à 12, présentant

    - une concentration de lactulose comprise entre 10 g/L et 50 g/L,
    - une concentration de lactose comprise entre 10 g/L et 100 g/L, et
    - une concentration de fructose < 45 g/L.

# Figur 1

Figur 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2008037839 A1
- US 8241658 B2
- US 9579340 B2
- US 7842678 B2
- WO 2014141164 A1

**In der Beschreibung aufgeführte Nicht-Patentliteratur**

- **WANG HE et al.** Enzymatic production of lactulose and 1-lactulose: current state and perspectives. *Appl Microbiol Biotechnol,* 2013, vol. 97, 6167-6180